# EUROPEAN PATENT APPLICATION

(11) **EP 2 301 548 A1**
(43) Date of publication of application: **30.03.2011**
(21) Application number: 10014149.8
(22) Date of filing: 31.03.2006
(51) Int. Cl.: A61K 31/555, A61K 9/08, A61P 1/16

(54) **Stannsoporfin compositions and administration**

(30) Priority: 01.04.2005 US 96359; 30.09.2005 US 239769
(62) Divisional of application: 06749109.2
(71) Applicant: InfaCare Pharmaceutical Corporation, Trevose PA 19053-6944 (US)
(72) Inventor: Lang, Philip C., Toms River New Jersey 08757 (US); Drummond, George, S., New York New York 10023 (US)
(74) Representative: Schmidt, Karsten

(57) **Abstract**

Pharmaceutical compositions including stannsoporfin, drug products incorporating pharmaceutical compositions, methods of making pharmaceutical composition, and methods of treating hyperbilirubinemia with drug products and compositions are disclosed.

## Description

### TECHNICAL FIELD

Tin (IV) mesoporphyrin IX dichloride, or stannsoporfin, is a mesoporphyrin chemical compound having the following structure:

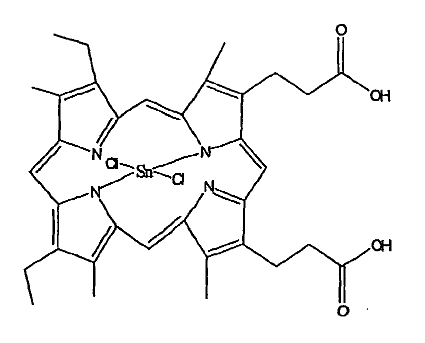

Stannsoporfin has been proposed for use, for example, as a medicament in the treatment of various diseases including, for example, psoriasis (U.S. Patent No. 4,782,049 to Kappas et al.) and infant jaundice (for example, in U.S. Patent Nos. 4,684,637, 4,657,902 and 4,692,440). Stannsoporfin is also known to inhibit heme metabolism in mammals, to control the rate of tryptophan metabolism in mammals, and to increase the rate at which heme is excreted by mammals (U.S. Patent Nos. 4,657,902 and 4,692,440).

Processes for obtaining stannsoporfin are known in the art. Protoporphyrin IX iron (III) chloride or hemin, of the structural formula: 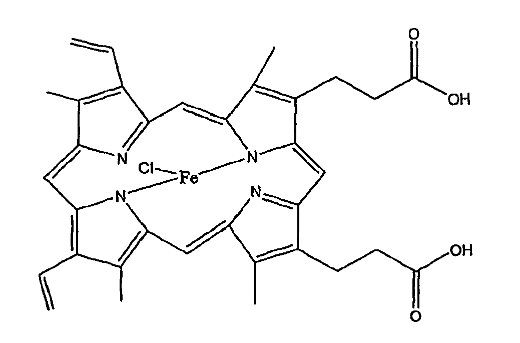 is commonly used as starting material. The hemin is generally hydrogenated to form an intermediate mesoporphyrin IX dihydrochloride, which is subsequently subjected to tin insertion, yielding stannsoporfin. Methods for making stannsoporfin are disclosed in United States Patent No. 6,818,763 and United States Patent Application Serial No. 10/812,156, filed on March 29, 2004, the contents of both of which are incorporated herein by reference.

One way of administering stannsoporfin is by an injectable solution. Although stannsoporfin has been provided in aqueous solutions in the past, it has been found that in stannsoporfin solutions having higher concentrations, the stannsoporfin does not adequately dissolve in the solution. It would be desirable to provide compositions, drug products and methods of manufacture that can include higher concentrations of stannsoporfin. It would further be desirable to provide compositions and drug products that include stannsoporfin that are stable and have an acceptable shelf life.

### DISCLOSURE OF THE INVENTION

One aspect of the present invention is directed towards a pharmaceutical composition or drug product comprising stannsoporfin in an aqueous solution, wherein the concentration of stannsoporfin in the solution is at least about 4 mg/ml, preferably at least about 4.5 mg/ml, and more preferably at least about 5 mg/ml. In one embodiment a preferred range of stannsoporfin concentration is between about 5-40 mg/ml, including concentrations of 10 mg/ml, 15 mg/ml, 20 mg/ml, 25 mg/ml, 30 mg/ml, 35 mg/ml and 40 mg/ml.

According to one embodiment, the composition or drug product has a physiological osmolarity. According to another embodiment, the composition or drug product is stable at room temperature for at least about one month. In other embodiments, the composition or drug product is stable at room temperature up to at least about two months, in still other embodiments, the solution or drug product is stable at room temperature up to at least about three months, and in other embodiments, the solution or drug product is stable at room temperature up to at least about six months. As used herein, room temperature includes, but is not limited to temperatures between about 68° F and 77° F.

Another aspect of the present invention is directed towards a drug product including a stannsoporfin solution, wherein the drug product includes a single dose of stannsoporfin in solution. In certain embodiments, the solution may further comprise a base, an acid and a buffering agent.

Another aspect of the present invention is directed towards a method of making a pharmaceutical composition comprising a stannsoporfin aqueous solution, wherein stannsoporfin is mixed with a buffering agent. The stannsoporfin is then dissolved through the addition of a base which preferably raises the pH to at least about 10. After the stannsoporfin has completely dissolved, the pH is then lowered within a physiological pH range by the addition of an acid so that it can be administered to a patient. As used herein, physiological pH range refers to a pH range of less than about 8. According to one or more embodiments as used herein, physiological pH range means a pH between about 7.2 and 7.9, and more preferably, a physiological pH means a pH between about 7.4 and 7.9.

Another aspect of this invention is directed towards a method of making a drug product comprising an aqueous solution of stannsoporfin in a concentration of at least about 4.5 mg/ml. Still another aspect of the invention pertains to treatment of hyperbilirubinemia utilizing the compositions and drug products disclosed herein.

The invention further resides in the following:
1. A pharmaceutical composition comprising stannsoporfin in an aqueous solution at a concentration of at least about 20 mg/ml and having a physiological osmolarity.
2. A pharmaceutical composition comprising stannsoporfin in an aqueous solution at a concentration of at least about 20 mg/ml and having a shelf life at room temperature of at least about 3 months.
3. The pharmaceutical composition defined in #1, wherein the composition has a shelf life at room temperature of at least about 3 months.
4. The pharmaceutical composition defined in #1, wherein the composition has a shelf life at room temperature of at least about 6 months.
5. The pharmaceutical composition defined in #1, wherein the composition has an osmolarity of between about 270 and 328 mOsmol/L.
6. The pharmaceutical composition defined in #1, wherein the composition has an osmolality of between about 250 and 300 mOsmol/kg.
7. A drug product including the pharmaceutical composition defined in #1, in a single dose unit.
8. A method of making a pharmaceutical composition comprising:
   mixing a pre-determined amount of stannsoporfin with a buffering agent in aqueous solution;
   increasing the pH of the solution to a pH of at least about 10 to facilitate dissolution of the stannsoporfin in the solution; and
   decreasing the pH of the solution to a pH of less than or equal to about 8.
9. The method defined in #8 , wherein the pH of the stannsoporfin solution is decreased to between about 7.4 and 7.9.
10. The method defined in #8, wherein the buffering agent is selected from the group consisting of an alkali earth metal buffering agent, a calcium buffering agent, a magnesium buffering agent, an aluminum buffering agent, sodium bicarbonate, potassium bicarbonate, magnesium hydroxide, magnesium lactate, magnesium gluconate, magnesium oxide, magnesium aluminate, magnesium carbonate, magnesium silicate, magnesium citrate, aluminum hydroxide, aluminum hydroxide/magnesium carbonate, aluminum hydroxide/sodium bicarbonate coprecipitate, aluminum glycinate, aluminum magnesium hydroxide, aluminum phosphate, sodium citrate, calcium citrate, sodium tartrate, sodium acetate, sodium carbonate, sodium polyphosphate, sodium dihydrogen phosphate, potassium polyphosphate, sodium polyphosphate, potassium pyrophosphate, disodium hydrogenphosphate, tribasic sodium phosphate dodecahydrate, dipotassium hydrogen phosphate, trisodium phosphate, tripotassium phosphate, potassium carbonate, potassium metaphosphate, calcium acetate, calcium glycerophosphate, calcium chloride, calcium hydroxide, calcium lactate, calcium carbonate, calcium gluconate, calcium bicarbonate, sodium phosphate, potassium phosphate, calcium phosphate, magnesium phosphate, potassium citrate, trihydroxymethylaminomethane, an amino acid, an acid salt of an amino acid, and an alkali salt of an amino acid and combinations thereof.
11. The method defined in #8, wherein the pH is increased by the addition of a base selected from the group consisting of sodium hydroxide, potassium hydroxide, calcium hydroxide, ammonium hydroxide, 10% ethanolamine and magnesium hydroxide.
12. The method defined in #8, wherein the pH is lowered by the addition of hydrochloric acid.
13. A pharmaceutical composition made by the method defined in #8.
14. A method of lowering bilirubin levels in a mammal comprising parenterally administering to said mammal the pharmaceutical composition defined in #13.
15. The method defined in #14, wherein the mammal is a human.
16. The method defined in #15, wherein the human is an infant.
17. A method of lowering bilirubin levels in a mammal comprising:
   parenterally administering to said mammal the drug product
18. The method defined in #17, wherein the mammal is a human.
19. The method defined in #18, wherein the human is an infant.
20. A method for increasing the bioavailablity of stannsoporfin following intramuscular administration in a human patient comprising intramuscularly administering to said patient a pharmaceutical composition comprising stannsoporfin in an aqueous solution having a physiological osmolarity.
21. The method defined in # 20, wherein the osmolarity is between about 270 and 328 mOsmol/L.
22. The method defined in # 20, wherein the amount of stannsoporfin administered is at least about 20 mg.
23. The methoddefined in #20, wherein the concentration of stannsoporfin in the pharmaceutical composition is at least about 20 mg/ml.
24. The method defined in #20 wherein the urinary excretion of stannsoporfin is at least about 2-fold greater than the urinary excretion of stannsoporfin in an aqueous solution having a non-physiological osmolarity.
25. The method defined in #24, wherein the urinary excretion of stannsoporfin is at least about 4-fold greater than the urinary excretion of stannsoporfin in an aqueous solution having a non-physiological osmolarity.
26. The method defined in #25, wherein the non-physiological osmolarity is hyperosmolar.
27. The method defined in #26, wherein the hyperosmolar osmolarity is about 400 mOsmol/L.
28. The method defined in #25, wherein the urinary excretion of stannsoporfin is greater at between about 24 hrs and about 48 hrs following administration of the pharmaceutical composition.
29. The method defined in #20, wherein the pharmaceutical composition is made a process comprising:
   mixing a pre-determined amount of stannsoporfin with a buffering agent in aqueous solution;
   increasing the pH of the solution to at least about 10 by addition of a base; and
   decreasing the pH of the solution to at least about 8 by addition of an acid.
30. The method defined in #20, wherein the concentration of stannsoporfin in the pharmaceutical composition is at least about 20 mg/ml.

### BEST MODE FOR CARRYING OUT THE INVENTION

It is to be appreciated that the various process parameters described herein (by way of example only, temperature, time, and pressure) are approximations and may be varied, and certain steps may be performed in different order. Before describing several exemplary embodiments of the invention, it is to be understood that the invention is not limited to the details of construction or process steps set forth in the following description. The invention is capable of other embodiments and of being practiced or carried out in various ways.

In overview, one or more embodiments relate to compositions, drug products and methods of treatment using stannsoporfin. As used herein, tin (IV) mesoporphyrin IX dichloride includes tin 4⁺ mesoporphyrin IX dichloride and stannsoporfin. Tin (IV) mesoporphyrin IX dichloride can be obtained according to a variety of methods, for example, through the methods disclosed in United States Patent No. 6,818,763, and co-pending United States Patent Application Serial No. 10/812,156 (Publication No. 20040210048), which are incorporated herein by reference. However, it should be understood that other methods can be used to produce mesoporphyrin halides such as tin mesoporphyrin IX dichloride, and the present invention is not limited to a particular method of mesoporphyrin production.

For example, a two-stage hydrogenation process can be used to prepare tin mesoporphyrin. In the first stage, a reaction mixture of hemin and a hydrogenation catalyst are subjected to a first elevated temperature for a first period of time. The first stage temperature can be in the range of about 85-95°C and the period of time is at least about one hour, for example, between about 1-3 hours.

In the second stage of hydrogenation, the reaction mixture is cooled to a second temperature for a second period of time. For example, the second temperature can be in a range of about 45-50°C and hydrogenated for a second period of time of about 3-6 hours, in order to convert substantially all hemin (protoporphyrin IX iron (III) chloride) to mesoporphyrin IX formate. This second stage can also be conducted in the presence of formic acid. The same catalyst may be used as in the first step described above, so that the two stages of the process may be conducted in the same reactor. Optionally, a further charge of hydrogen may be supplied to the reactor prior to commencing the second stage. In some situations, the second hydrogenation stage increases the yield of the mesoporphyrin IX formate, while reducing the amount of impurities in the final metal mesoporphyrin halide. (See the following Figure).

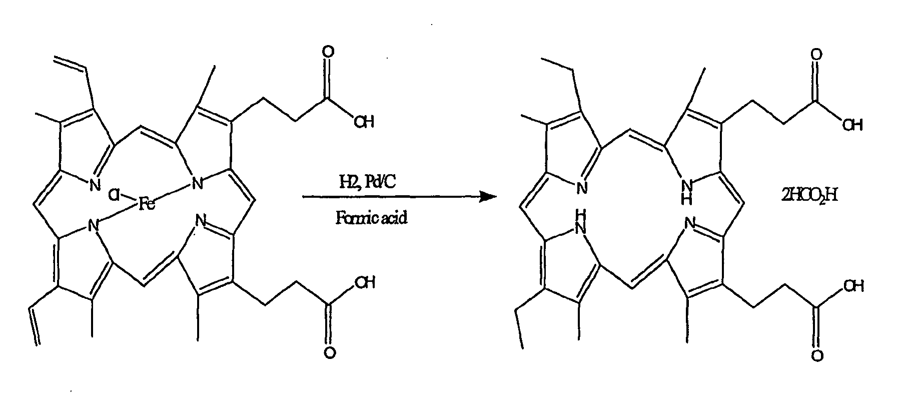

By the method described above, the mesoporphyrin IX intermediate compound in the present invention is not isolated as a dihydrochloride, but rather as a formate salt. It will be understood, of course, that other processes can be used for the preparation of tin (IV) mesoporphyrin intermediates.

The mesoporphyrin IX formate may be isolated from a formic acid solution by the addition of a solvent such as ether or another organic solvent, leading directly to the mesoporphyrin IX formate intermediate, which is further subjected to drying. Ethers such as, for example, methyl tert-butyl ether, diethyl ether or di-isopropyl ether, among others, may be used.

According to the process described above, less solvent is required compared to other processes, and such smaller volumes allow for less filter time to obtain the intermediate. Ratios of the amount of hemin to the amount of solvent of about 1:10 to about 1:20 may be used. In addition, the filtration and washings of the mesoporphyrin IX formate are rapid. After drying, a crude intermediate formate is obtained, in high yields (about 80-95%) and its purity, established by HPLC, is about or above 97%.

The insertion of metal into mesoporphyrin IX formate to obtain metal mesoporphyrin halide is described below with specific reference to tin, to prepare stannsoporfin.

The insertion of tin into mesoporphyrin IX formate is utilized to create tin mesoporphyrin. To create tin mesoporphyrin, mesoporphyrin IX formate is subjected to heating with a tin (II) carrier in an acid such as acetic acid, buffered with an acetate ion, in the presence of an oxidant, at reflux. Tin (II) carriers such as tin (II) halides or tin (II) acetate can be used. Suitable acetate counter ions include ammonium, sodium or potassium ions. Oxidants such as oxygen from air or in pure form as well as hydrogen peroxide can also be used. Mesoporphyrin IX formate can be subjected to heating with tin (II) chloride in acetic acid, buffered with ammonium acetate, and the reaction is conducted in the presence of air, at reflux. During this process, tin mesoporphyrin dichloride is isolated from the reaction mixture by the addition of water, followed by filtration to provide a filter cake. Prior to drying at about 90-100°C, the filter cake is triturated into hot, dilute hydrochloric acid, for example, at a concentration of about O.1N-6N, at about 90-100°C. The crude, substantially pure tin mesoporphyrin chloride (crude tin (IV) mesoporphyrin IX dichloride) is obtained with a yield of about 75-95% and a purity of about 95%, as judged by HPLC analysis. (See the following Figure).

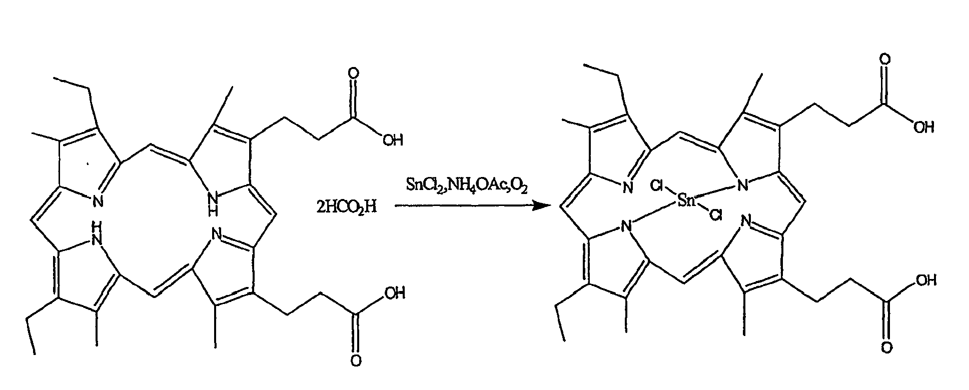

The tin mesoporphyrin IX dichloride obtained by the above-described process may be further purified by dissolving the product in an aqueous inorganic base solution, for example, dilute ammonium hydroxide, followed by treatment with charcoal. The product is then re-precipitated by addition to an acid solution, such as acetic acid, hydrochloric acid or a mixture thereof. The above dissolving, charcoal treatment and re-precipitation steps may be repeated a number of times, typically about 1-3 times in order to ensure the desired purity. Prior to drying, the cake is triturated in hot, dilute hydrochloric acid of a concentration of about 0.1N-6N, at a temperature of about 90-100°C, in order to remove any residual ammonium salts. The tin mesoporphyrin chloride product (tin (IV) mesoporphyrin IX dichloride or stannsoporfin) is obtained in a yield of about 50-70%, with an HPLC purity of about or greater than 97%.

The process described above may also be performed to produce substantially pure or pharmaceutical quality tin mesoporphyrin chloride (tin (IV) mesoporphyrin IX dichloride or stannsoporfin) in large scale quantities, such as quantities exceeding about 0.1kg through and including multiple kilogram amounts, by slight modifications of the above procedure, such as increased reaction or drying times as appropriate based upon the increase in scale of the starting reactants. Temperature and pressure times likewise can be modified as needed. The tin mesoporphyrin chloride product (tin (IV) mesoporphyrin IX dichloride or stannsoporfin) is obtained in the large scale production process in a yield of about 60-90%, with an HPLC purity of about 97%.

Alternatively, the stannsoporfin can be obtained by the methods disclosed in co-pending and United States Patent Application Serial No. 10/812,156, filed on March 29, 2004, the entire contents of which is incorporated herein by reference. In overview, stannsoporfin can be produced by isolating a mesoporphyrin formate and converting the mesoporphyrin formate to a tin mesoporphyrin halide. The mesoporphyrin formate can be converted directly to a metal mesoporphyrin halide, or alternatively, the mesoporphyrin formate can first be converted to mesoporphyrin dihydrochloride and the mesoporphyrin dihydrochloride can then be converted to the metal mesoporphyrin halide.

The compositions of the present invention can be prepared and administered in a wide variety of parenteral dosage forms. Thus, the compositions of the present invention can be administered by injection, that is, intravenously, intramuscularly, intrathecally, intracutaneously, subcutaneously, intraduodenally, or intraperitoneally. Additionally, the compositions of the present invention can be administered transdermally.

Liquid form preparations can include solutions, suspensions, and emulsions, for example, water or water propylene glycol solutions. For parenteral injection, liquid preparations can be formulated in solution in aqueous solutions as described herein.

The pharmaceutical preparation is preferably in unit dosage form. In such form, the preparation is subdivided into unit doses containing appropriate quantities of the active component. The unit dosage form can be a packaged preparation, the package containing discrete quantities of preparation, such as packeted in vials or ampules.

The quantity of active component in a unit dose preparation may be varied or adjusted from about 0.1 to about 50 mg, preferably 0.1 to about 40 mg, and more preferably 0.1 to about 20 mg according to the particular application and the potency of the active component and size of the patient. The composition can, if desired, also contain other compatible therapeutic agents.

In therapeutic use as agents for treating neonatal hyperbilirubinemia, the compounds utilized in the pharmaceutical methods of this invention are administered at the initial dosage of about 0.1 mg to about 20 mg per kilogram body weight (IM) daily. Specific exemplary embodiments involve the use of about 0.5 mg to about 6 mg per kilogram body weight (IM) for the treatment of neonatal hyperbilirubinemia. The dosages, however, may be varied depending upon the requirements of the patient, the severity of the condition being treated and the compound being employed. Determination of the proper dosage for a particular situation is within the skill of the art. In one embodiment, generally, treatment is initiated with smaller dosages which are less than the optimum dose of the compound. Thereafter, the dosage is increased by small increments until the optimum effect under the circumstance is reached.

One aspect of the present invention is directed towards a pharmaceutical composition that comprises stannsoporfin, where it is in an aqueous solution and the concentration of stannsoporfin in the solution is between about 4.5 and 40 mg/ml, and preferably between about 4.5 and 25 mg/ml. In one or more embodiments, the components of the stannsoporfin composition comprise an acid, a base and a buffering agent mixed in an aqueous solution. The composition is preferably sterile and has a physiological osmolarlity. The compositions or drug products are preferably packaged in amber glass vials.

The pharmaceutical composition containing stannsoporfin can be a component of a drug product, wherein the product is contained in a single dose unit. According to one embodiment, a single dose unit includes at least about 0.5 ml of solution, and more preferably, at least about 1 ml of solution.

The solution may be provided in a drug product form by containing the solution in a suitable container such as an ampule or vial. According to certain embodiments, the solution is stable and has a shelf life of at least about 3 months. In other embodiments, the solution has a shelf life of at least about 6 months.

Another aspect of this invention is directed towards a method of making a pharmaceutical composition comprising large quantities of stannsoporfin. In one or more embodiments, the stannsoporfin is present in an amount of at least about 4.5 mg/ml. In an exemplary embodiment, a pre-determined amount of stannsoporfin is mixed with a buffering agent in aqueous solution. There are numerous buffers which may be suitable for creating the pharmaceutical composition. Examples of such buffers include: an alkali earth metal buffering agent, a calcium buffering agent, a magnesium buffering agent, an aluminum buffering agent, sodium bicarbonate, potassium bicarbonate, magnesium hydroxide, magnesium lactate, magnesium gluconate, magnesium oxide, magnesium aluminate, magnesium carbonate, magnesium silicate, magnesium citrate, aluminum hydroxide, aluminum hydroxide/magnesium carbonate, aluminum hydroxide/sodium bicarbonate coprecipitate, aluminum glycinate, aluminum magnesium hydroxide, aluminum phosphate, sodium citrate, calcium citrate, sodium tartrate, sodium acetate, sodium carbonate, sodium polyphosphate, sodium dihydrogen phosphate, potassium pyrophosphate, sodium polyphosphate, potassium pyrophosphate, disodium hydrogenphosphate, tribasic sodium phosphate dodecahydrate, dipotassium hydrogen phosphate, trisodium phosphate, tripotassium phosphate, potassium carbonate, potassium metaphosphate, calcium acetate, calcium glycerophosphate, calcium chloride, calcium hydroxide, calcium lactate, calcium carbonate, calcium gluconate, calcium bicarbonate, sodium phosphate, potassium phosphate, calcium phosphate, magnesium phosphate, potassium citrate, trihydroxymethylaminomethane, an amino acid, an acid salt of an amino acid, and an alkali salt of an amino acid, and combinations of the foregoing. The buffer used should be able to be used in a concentration effective to raise the pH of the solution to about 10 or above, when base is added to the solution. In addition, the buffer must be pharmaceutically acceptable.

According to one or more embodiments, the method of making a pharmaceutical composition further comprises adjusting the pH of the solution to a pH of at least about 10 to facilitate dissolution of the stannsoporfin in the solution. This can be accomplished through the addition of a strong base to the solution. Strong bases with low pK_{b} values facilitate the dissolution of large quantities of stannsoporfin. The base used in this method can be any pharmaceutically acceptable strong base such as a metal hydroxide or other hydroxide bases. Presently preferred bases include pharmaceutically acceptable Group I and Group II metal hydroxides. Sodium hydroxide has been demonstrated to be suitable. Other suitable bases may include potassium hydroxide, calcium hydroxide, ammonium hydroxide, tetraethyl ammonium hydroxide, 10% ethanolamine or magnesium hydroxide. The base should be pharmaceutically acceptable and effective to raise the pH of the solution to about 10 or above.

According to one or more embodiments of the method of the invention the pH of the solution is adjusted to a pH range of less than 8, for example between about 7.2 and 7.9, more preferably to a pH of between about 7.4 to 7.9. This can be accomplished through the addition of a pharmaceutically effective strong acid in a dilute concentration to the solution corresponding to the base used to raise the pH. One example of suitable acid is 0.3 N. hydrochloric acid. Other suitable acids may include niric acid, perchloric acid or hydroiodic acid. In preferred embodiments, the pH range of the stannsoporfin solution should be in a form that can be administered so the pH range is preferably between about 7.4 and 7.9.

Another aspect of this invention is directed towards a method of lowering bilirubin levels in a mammal comprising parenterally administering a stannsoporfin solution that has a concentration of stannsoporfin greater than 4.5 mg/ml. While the intended recipients of this medication to treat hyperbilirubinemia are humans, particularly infants, the stannsoporfin solution may also be effective in other mammals.

Another aspect of this invention is directed towards a method of making a drug product comprised of an aqueous solution of stannsoporfin in a concentration of at least about 4.5 mg/ml. This aqueous solution can further comprise an acid, a base and a buffering agent. One way in which this aqueous solution can be packaged is in a vial. Preferably, the vial is a Type 1 amber glass tubing vial to protect the composition from light. According to one or more embodiments, the drug product is stable and has a shelf life of at least about 1 month, and preferably at least about 3 months. In other embodiments, the product has a shelf life of at least about 6 months at room temperature.

In certain embodiments, the drug product or compositions exhibits a physiological osmolarity. As used herein, the phrase "physiological osmolarity" means the drug product or composition, when administered to a patient does not cause irritation or an adverse reaction. Previous formulations did not exhibit a physiological osmolarity, and administration of the composition caused irritation to the patient. A suitable range for the osmolarity according to certain embodiments is between about 270 and 328 mOsmol/L, and more preferably between about 280-300 mOsmol/L osmolarity.

Exemplary embodiments of the invention will be further described for illustrative purposes with reference to the following non-limiting examples.

### EXAMPLE 1

### PREPARATION OF AN AQUEOUS SOLUTION OF STANNSOPORFIN

A desired volume of the batch for the solution was determined. The amount of stannsoporfin required for the batch was calculated and recorded. The quantities of base (for example, 1N sodium hydroxide, NaOH) and acid (for example, 0.3N hydrochloric acid, HCl) required for adjusting the pH of the batch were calculated and recorded. The projected final weight of the solution batch based on the components and based on the theoretical density of the stannsoporfin injection was calculated and recorded.

An empty mixing vessel was purged with nitrogen NF for a minimum of 15 minutes. Purging continued throughout the formulation process. Water for injection was added to the vessel. The temperature of the mixing vessel was measured and adjusted to between about 15°C to 30°C. The mixing vessel was maintained within this temperature range throughout the process. Mixing began at 400 to 600 rpm, and the mixing was maintained at this rate throughout the process. A buffering agent (for example, tribasic sodium phosphate dodecahydrate) was added and the solution was mixed for 30 to 35 minutes, until the buffering agent completely dissolved. Stannsoporfin was added to the mixing vessel. The contents of the vessel were now mixed for approximately 30 to 35 minutes. A 10 ml sample of the bulk solution was withdrawn and its pH was measured. If the pH was below about 10, small increments of 1N sodium hydroxide solution was added to aid in the dissolution of the stannsoporfin.

The pH was increased to above about 10 and any amount of added sodium hydroxide was recorded. The solution was then observed to determine if the stannsoporfin was dissolved. The contents of the vessel were mixed for approximately 30 to 35 minutes. Another 10 ml sample of the bulk solution was withdrawn, and its pH was measured.

If the pH was above about 8, it was adjusted downwards by adding small increments of 0.3N hydrochloric acid solution. Any amount of acid added was recorded. The amount of water for injection to add to obtain the final weight of the solution was determined. The water for injection was added until the final weight of the solution was reached. The contents of the vessel were then mixed for ≈ 45 to 50 minutes. A 10 ml sample was withdrawn and its final pH was recorded. The final weight of the solution was also measured.

### EXAMPLE 2

### A STANNSPORFIN SOLUTION

A 23 L stannsoporfin solution was prepared in accordance with Example 1 including the following components:

| Component | Amount/ml | Amount/23L |
|---|---|---|
| Stannsoporfin | 0.020g | 460.0g |
| Tribasic Sodium Phosphate Dodecahydrate, ACS | 0.017g | 391.0g |
| Sodium Hydroxide NF (1*N* solution) | 0.090ml | 2.0L |
| Hydrochloric Acid NF (0.3*N* solution) | 0.090ml | 2.0L |
| Water for Injection USP | qs to 1.0ml | qs to 23.0L |
| Nitrogen NF | qs headspace | qs headspace |

### EXAMPLE 3

### STANNSOPORFIN SOLUTION STABILITY TESTS

Samples produced in accordance with EXAMPLES 1 and 2 were subjected to stability testing as follows. The stability samples were stored at 3 conditions (4°C ± 2°C, 25°C ± 2°C/60% ± 5%RH and, 40°C ± 2°C/75% ± 5%RH), and were monitored as follows: 4°C ± 2°C for 0, 3, 6, 9, 12, 18, 24, 36, 48, and 60 months; 25°C ± 2°C/60% ± 5%RH for 0, 3, 6, 9, 12, 18, 24, 36, 48, and 60 months; 40°C ± 2°C/75% ± 5%RH 0, 1, 2, 3, 4, 5, and 6 months. At the indicated time intervals for each storage condition, the stability samples were tested for appearance, assay, purity, pH, identification, and sterility. Appearance, Peak Purity [by diode array detector (DAD)], Identity (UV), and sterility according to USP were satisfactory at each test point, unless otherwise indicated. Sterility testing was performed initially and at 12 and 24 months.

Limits:
pH: 7.4 to 8.0
Volume in Container: Not less than 1.5 ml per vial withdrawable.
Particulate Matter (USP <877>):
   NMT 6,000 parts ≥ 10 µm per vial.
   NMT 600 parts ≥ 25 µm per vial.
   NMT= Not More Than
Assay: 95.0% to 105.0% of label
Impurities (total): NMT 3%
Sterility (USP <71>): Sterile
Bacterial Endotoxins (USP <85>): Contains NMT 0.7 EU/mg of Stannsoporfin Injection
EU= Endotoxin Units
All measured parameters were within acceptable limits.

### EXAMPLE 4

### OSMOLARITY OF A STANNSOPORFIN SOLUTION

A formulation was prepared in accordance with the having the following components therein:

| Component | Amount/ml | Amount/25ml |
|---|---|---|
| 1. Stannsoporfin | 20.02 mg | 500.5 mg |
| 2. Tribasic Sodium Phosphate Dodecahydrate, ACS | 16.0 mg | 400.2 mg |
| 3. Reverse Osmosis Water | Q.S. to 75% of Final Vol. | 19 ml |
| 4. Sodium Hydroxide NF (1*N* solution) | titrate to | 500 µl |
| | pH > 10 | |
| 5. Hydrochloric Acid NF (0.3N solution) titrate to | | |
| | pH 7.4-7.8 | 900 µl |
| 6. Reverse Osmosis Water | Q.S. to Final volume | |

Components 1, 2 and 3 were mixed together, and then component 4 was added to titrate the solution to a pH of about 10.56. Component 5 was then added to titrate the solution to a pH of 7.62. Water was added Q.S. to volume.

Osmolality was measured using a Wescore 5500 Vapor Pressure Osmometer. Prior to measuring the osmolality of the samples, the osmometer was calibrated using osmolality standards of 290 mmol/kg, 1000 mmol/kg and 100 mmol/kg. The osmolality values were 255, 252 and 256 mmol/kg, and the average value was 254 mmol/kg.

### EXAMPLE 5

### OSMOLARITY OF A STANNSOPORFIN SOLUTION

A formulation was prepared having the following components therein:

| Component | Amount/ml | Amount/25ml |
|---|---|---|
| 1. Stannsoporfin | 20.03 mg | 500.7 mg |
| 2. Tribasic Sodium Phosphate Dodecahydrate, ACS | 17.0 mg | 425 mg |
| 3. Reverse Osmosis Water | Q.S. to 75% of Final Vol. | 19 ml |
| 4. Sodium Hydroxide NF (1*N* solution) | titrate to pH > 10 | 1 ml |
| 5. Hydrochloric Acid NF (0.3*N* solution) | titrate to pH 7.4-7.8 | 1 ml |
| 6. Reverse Osmosis Water | Q.S. to Final volume | |

Components 1, 2 and 3 were mixed together, and then component 4 was added to titrate the solution to a pH of about 10.56. Component 5 was then added to titrate the solution to a pH of 7.62. Water was added Q.S. to volume.

Osmolality was measured using a Wescore 5500 Vapor Pressure Osmometer. Prior to measuring the osmolality of the samples, the osmometer was calibrated using osmolality standards of 290 mmol/kg, 1000 mmol/kg and 100 mmol/kg. The osmolality values were 292, 289 and 284 mmol/kg, and the average value was 288 mmol/kg.

### EXAMPLE 6

### URINARY EXCRETION OF STANNSOPORFIN SOLUTIONS

Urinary excretion of two stannsoporfin formulations differing in osmolarity was measured as a marker of drug absorption from the muscle compartment into the central circulatory compartment. Forty mg of a prior art formulation having a non-physiological osmolarity of 400 mOsmol/L or a formulation having a physiological osmolarity of 270-328 mOsmol/L prepared according to the present invention were administered intramuscularly to healthy adult volunteers. Urine samples were collected up to 48 hrs after administration and excreted tin mesoporphyrin measured fluorometrically and expressed as % of dose administered.

| Formulation | Prior Art | Present Invention |
|---|---|---|
| Osmolarity (mOsmol/L) | 400 | 270-328 |
| Urine Excretion (% of Dose) | 0.20-0.38 | 0.79-8.35 |

No urinary tin mesoporphyrin was detected after 24 hrs following administration of the non-physiologic stannsoporfin formulation, whereas urinary tin mesoporphyrin was detected in urine up to and including 48 hrs following administration of the physiologic stannsoporfin formulation. The data suggest greater drug absorption of stannsoporfin into the circulatory system from the formulation having a physiologic osmolarity as compared to the formulation that is somewhat hyperosmolar, thereby indicating an increase the drug's bioavailablity.

While the foregoing is directed to various embodiments of the present invention, other and further embodiments of the invention may be devised without departing from the basic scope thereof, and the scope thereof is determined by the claims that follow.

## Claims

1. A pharmaceutical composition comprising stannsoporfin in an aqueous solution at a concentration of at least about 20 mg/ml and having a physiological osmolarity of between about 270 to about 328 mOsmol/L, wherein the aqueous solution includes a strong acid in a dilute concentration, a strong base with a low pK_{b} value and a buffering agent.

2. The pharmaceutical composition of claim 1, wherein the composition has a shelf life at room temperature of at least about 3 months.

3. The pharmaceutical composition of any one of claims 1 to 2, wherein the composition has an osmolality of between about 270 and 300 mOsmol/kg.

4. The composition of any one of claims 1 to 3 for use as a medicament.

5. The composition of claim 4, wherein said medicament is for lowering bilirubin levels in a mammal, and wherein said medicament is formulated for parenteral administration.

6. The composition of claim 4, wherein said medicament is for increasing bioavailability of stannsoporfin, wherein the medicament is formulated for intramuscular administration and wherein said bioavailability is measured as an increase in urinary excretion of stannsoporfin when compared to urinary excretion of stannsoporfin after intramuscular administration of a formulation of stannsoporfin having a non-physiological osmolarity.

7. The composition of claim 6, wherein the urinary excretion of stannsoporfin is at least about 2-fold greater than the urinary excretion of stannsoporfin in an aqueous solution having a non-physiological osmolarity.

8. The composition of claim 6, wherein the non-physiological osmolarity is hyperosmolar.

9. The composition of claim 8, wherein the hyperosmolar osmolarity is about 400 mOsmol/L.

10. The composition of claim 6, wherein the urinary excretion of stannsoporfin is greater at between about 24 hrs and about 48 hrs following administration of the medicament.

11. A method of making a pharmaceutical composition or drug product of any one of claims 1 to 3 comprising: mixing a pre-determined amount of stannsoporfin with a buffering agent in aqueous solution; increasing the pH of the solution to a pH of at least about 10 to facilitate dissolution of the stannsoporfin in the solution; and decreasing the pH of the solution to a pH of less than or equal to about 8.

12. The method of claim 11, wherein the pH of the stannsoporfin solution is decreased to between about 7.4 and 7.9.

13. The method of claim 11, wherein the buffering agent is selected from the group consisting of an alkali earth metal buffering agent, a calcium buffering agent, a magnesium buffering agent, an aluminum buffering agent, sodium bicarbonate, potassium bicarbonate, magnesium hydroxide, magnesium lactate, magnesium gluconate, magnesium oxide, magnesium aluminate, magnesium carbonate, magnesium silicate, magnesium citrate, aluminum hydroxide, aluminum hydroxide/magnesium carbonate, aluminum hydroxide/sodium bicarbonate coprecipitate, aluminum glycinate, aluminum magnesium hydroxide, aluminum phosphate, sodium citrate, calcium citrate, sodium tartrate, sodium acetate, sodium carbonate, sodium polyphosphate, sodium dihydrogen phosphate, potassium polyphosphate, sodium polyphosphate, potassium pyrophosphate, disodium hydrogenphosphate, tribasic sodium phosphate dodecahydrate, dipotassium hydrogen phosphate, trisodium phosphate, tripotassium phosphate, potassium carbonate, potassium metaphosphate, calcium acetate, calcium glycerophosphate, calcium chloride, calcium hydroxide, calcium lactate, calcium carbonate, calcium gluconate, calcium bicarbonate, sodium phosphate, potassium phosphate, calcium phosphate, magnesium phosphate, potassium citrate, trihydroxymethylaminomethane, an amino acid, an acid salt of an amino acid, and an alkali salt of an amino acid and combinations thereof.

14. The method of claim 11, wherein the pH is increased by the addition of a base selected from the group consisting of sodium hydroxide, potassium hydroxide, calcium hydroxide, ammonium hydroxide, 10% ethanolamine and magnesium hydroxide.

15. The method of claim 11, wherein the pH is lowered by the addition of hydrochloric acid.
